# EUROPEAN PATENT SPECIFICATION

(11) **EP 2 386 544 B1**
(45) Date of publication and mention of the grant of the patent: **10.02.2016**
(21) Application number: 10162298.3
(22) Date of filing: 07.05.2010
(51) Int. Cl.: C07D 211/90

(54) **Preparation of dihydropyridines**
Herstellung von Dihydropyridinen
Préparation of dihydropyridines

(43) Date of publication of application: 16.11.2011
(60) Divisional application: 15200796.9
(73) Proprietor: Laboratorios Lesvi, S.L., 08970 Sant Joan Despí - Barcelona (ES)
(72) Inventor: Vishnu Newadkar, Ravindranath, 400701, Mahape, Navi Mumbai (IN); Puruschottam Joshi, Anil, 400701, Mahape, Navi Mumbai (IN); Kumar Singh, Santosh, 400701, Mahape, Navi Mumbai (IN)
(74) Representative: Pons Ariño, Angel

(56) References cited:
- WO-A1-95/12578
- DATABASE chemcats [Online] 23 September 2009 (2009-09-23), XP002601964 Database accession no. 2051161626 & 23 September 2009 (2009-09-23), APAC pharmaceutical Product List , APAC- Pharmaceutical, LLC 6851- Oak Hall Lane, Suite 101, Columbia, MD, 21045 USA RN 1020718-27-7
- DATABASE CA [Online] CHEMICAL ABSTRACTS SERVICE, COLUMBUS, OHIO, US; 2002, STROHMEIER, GERNOT A. ET AL: "Rapid Parallel Synthesis of Polymer-Bound Enones Utilizing Microwave-Assisted Solid-Phase Chemistry" XP002601962 retrieved from STN Database accession no. 2002:35334 -& JOURNAL OF COMBINATORIAL CHEMISTRY , 4(2), 154-161 CODEN: JCCHFF; ISSN: 1520-4766, 2002, XP002601963
- ALAJARIN R. ET AL.: "Synthesis of unsimmetrically substituted 1,4-dihydropyridines and analogous calcium antagonistrs by microwave heating" SYNTHESIS, 1995, pages 389-391, XP002601965
- LEONARDI A. ET AL.: "Asymmetric N-(3,3-diphenylpropyl)aminoalkyl esters of 4-aryl-2,6-dimethyl-3,5,dicarboxylic acids with antihypertensive activity" EUROPEAN JOURNAL OF MEDICINAL CHEMISTRY, vol. 33, 1998, pages 399-420, XP002601966

## Description

### FIELD OF THE INVENTION

This invention relates to an improved method for the preparation of clevidipine, also known as clevidipine butyrate, a very short acting hypertensive calcium antagonist, as well as the synthesis of new compounds useful for the preparation of clevidipine.

### BACKGROUND OF THE INVENTION

Clevidipine butyrate is a dihydropyridine calcium channel blocker, currently indicated for the reduction of blood pressure, when oral therapy is not feasible or not desirable. Its chemical name is butyroxymethyl 4-(2,3-dichlorophenyl)-2,6-dimethyl-1,4-dihydropyridine-3,5-dicarboxylate and its chemical structure is depicted below:

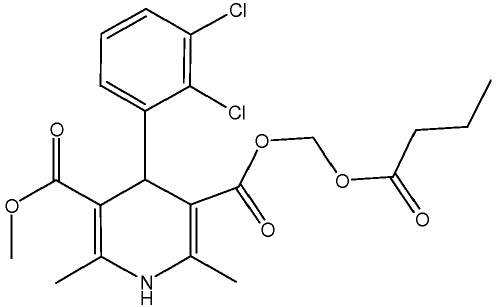

It is commercialized under the brand name of Cleviprex®, as a racemic mixture, in which each enantiomer has antihypertensive activity. Cleviprex® is an emulsion, suitable for intravenous applications.

Clevidipine butyrate was first described in WO9512578, as a very useful short-acting steerable antihypertensive drug, for intravenous administration.

WO9512578 discloses a method for preparing clevidipine butyrate from 1,4-dihydro-2,6-dimethyl-4-(2',3'-dichlorophenyl)-5-carboxymethyl-3-pyridinecarboxylic acid and chloromethyl butyrate. This route has several drawbacks. Firstly, the purification of the final product is carried out by chromatographic methods, which are generally expensive, environmentally unfriendly and time consuming, therefore not recommended for industrial application. A second negative aspect is the nature of the solvent used. WO9512578 teaches that the reaction must be carried out in DMF or acetonitrile, both polar aprotic solvents, therefore reducing significantly the number of industrially suitable solvents. For instance, acetonitrile, solvent of used in patent application WO0031035, has suffered a worldwide commercial shortage over the last few years. Moreover, DMF, a solvent considered as a possible carcinogen by the International Agency for Research on Cancer (IARC), has been linked to cancer in humans and to cause birth defects.

Additionally, the present inventors have found that in order to achieve the purity requirements needed for an injectable formulation, the product as obtained in the WO '578 would have to be purified further.

WO0031035 tries to reduce some of the drawbacks associated to the process disclosed in the WO'578. The improvement is related to the use of the sodium and potassium salt of the 4-(2',3'-dichlorophenyl)-1,4-dihydro-5-methoxycarbonyl-2,6-dimethyl-3-pyridinecarboxylate, instead of the acid form previously described. The improved process is depicted below:

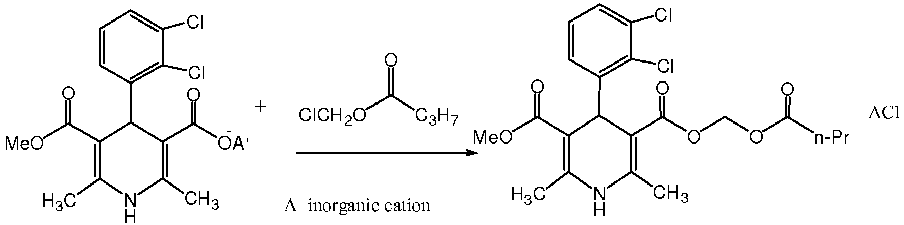

Although said process offers some improvements over the prior art, it still has some limitations. Similarly to WO '578, chloromethyl butyrate is also used in the last reaction step. According to the material safety data sheet of chloromethyl butyrate, it is corrosive, flammable and it can cause eye burns, skin burns, gastrointestinal tract burns and chemical burns to the respiratory tract. Therefore, its use increases the production costs as it is necessary special equipments and manufacturing facilities. The use of chloromethyl butyrate should be avoided in the last steps of synthetic processes, where the equipment is more expensive. The excess use of chloromethyl butyrate would lead to the generation of additional impurities similar in nature to the final product. It is well known in the art, that the more similar two compounds are, the more difficult the purification is. Consequently, clevidipine butyrate, directly obtained by the processes disclosed in WO '035, would have to be further purified in order to be used in the manufacture of pharmaceutical compositions. This mandatory extra purification step is time consuming and difficult to be carried out on industrial scale. In any case, this approach will lead to the formation of at least one equivalent of sodium chloride, which will have to be removed.

Equally to WO '578, the last step to obtain clevidipine is limited to the use of a polar aprotic solvent reducing significantly the number of industrially suitable solvents. Since the last reaction step takes place in acetonitrile and being clevidipine butyrate soluble in said solvent, a solvent- exchange must be performed in order to isolate clevidipine butyrate in a solid form from the reaction media.

Moreover, the process disclosed in the WO '578 implies the use of the cyanide intermediate, cyanoethyl methyl 4-(2',3'-dichlorophenyl)-2,6-dimetyl-1,4-dihydropyridine-3,5-dicarboxylate, to obtain the dihydropirydine carboxylate salt. As it is well known in the art, many cyanide-containing compounds are toxic, since they can readily release hydrogen cyanide (HCN) or cyanide ions. Both, HCN or the cyanide ions, are highly toxic to animals and human beings. The cyanide group is introduced as a protecting group. This means that the use of the cyanide intermediate leads to an increase of the number of manufacturing steps (protection followed by deprotection and purification) to prepare clevidipine butyrate. Although the yield disclosed for the last synthetic steps is relatively high, as a result of the increased number of manufacturing steps, the overall yield will not be that high when the overall synthesis (starting from the raw materials) is taken into consideration.

In accordance with health registration requirements of the U.S. and international health registration authorities, e.g. the FDA's Good Manufacturing Practices ("GMP") requirements, when preparing pharmaceutical compositions containing clevidipine butyrate for administration to mammals, there is a need to produce crystalline forms, or polymorphs, of clevidipine butyrate as pure as possible. Especially important are those forms, which have constant physical properties. Although those differences disappear once the compound is dissolved, they can appreciably influence pharmaceutically relevant properties of the solid form, such as handling properties, dissolution rate and stability. Such properties can significantly influence the processing, shelf life, and commercial acceptance of a polymorph.

### SUMMARY OF THE INVENTION

The present invention provides a process for the synthesis of clevidipine butyrate wherein the clevidipine butyrate is directly obtained in a high purity and yield. The process for the preparation of clevidipine butyrate comprises the reaction of methyl 3-amino crotonate with benzylidine butyrate, compound of formula (I): 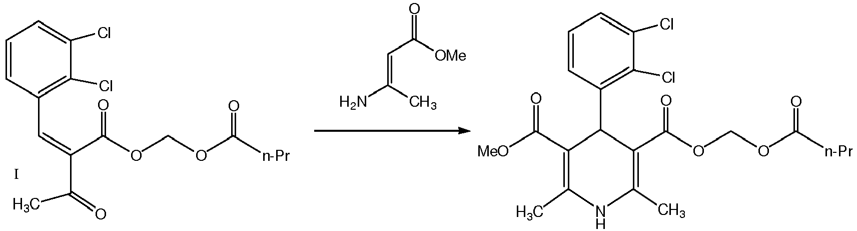 in the presence of at least an organic solvent or a solvent mixture comprising an organic solvent, wherein compound (I) is prepared by reacting 2-acetyl-3-(2',3'-dichlorophenyl)prop-2-enoic acid (compound (II)) with L-CH2-OCO-nPr. The present process is versatile enough, and it is not restricted to a specific kind of solvents. Therefore, if desired it is possible to carry out the reaction in a solvent where the clevidipine could be directly crystallised from, or in a solvent characterised by its low toxicity properties. The necessary starting materials for the last steps and co-reactants are non-toxic and they are either commercial or obtained with less synthetic steps compared with the processes disclosed in the art. The impurities identified for the process are easily removed by the techniques commonly used at industrial scale.

Disclosed are also compounds 2-(2',3'-dichloro-benzylidene)-3-oxo-butyric acid and 2-(2',3'-dichloro-benzylidene)-3-oxo-butyric acid butyryloxymethyl ester and their preparation as well as their use as intermediates for the preparation of clevidipine butyrate. These intermediate compounds are obtained in high purity and yield and can be easily isolated and purified by using standard industrial methods, avoiding the use of column chromatography. Moreover, the process of the invention permits the isolation of some of the intermediates, which is a clear advantage, as it is explained below.

Also disclosed are crystalline forms of clevidipine butyrate and their method for preparing thereof, useful to improve the pharmaceutical performance of pharmaceutical products.

### BRIEF DESCRIPTION OF THE DRAWINGS

Fig 1: XRD of clevidipine butyrate form A.
Fig 2: FTIR of clevidipine butyrate form A.
Fig 3: XRD of clevidipine butyrate form B.
Fig 4: FTIR of clevidipine butyrate form B.

### Definitions

The term "leaving group" refers to a chemical group capable of being displaced by a nucleophile. The structure of the leaving group will depend, in part, on the general reaction conditions employed such as structure of the nucleophile, solvent, temperature and time, all within the knowledge and control of the skilled artisan. Examples of leaving groups commonly employed include sulfonyl esters such as trifluoromethylsulfonyl, para-nitrobenzenesulfonyl, para-toluenesulfonyl, methylsulfonyl and the like; carboxyl esters such as trifluoroacetyl, para-nitrobenzoyl, para-methylbenzoyl, acetyl and the like; and halogens such as iodo, bromo, chloro, fluoro and the like. In J. March Advanced Organic Chemistry, 4th edition, 1992, are listed some typical leaving groups. In the context of the present invention, the leaving groups are preferably selected from halogens and sulphonyloxy groups. The halogens include fluorine, chlorine, bromine and iodine. The most preferred halogen is chlorine. The sulphonyloxy group is represented by -OSO₂R₂, wherein R₂ is a substituted or un-substituted linear or branched C₁-C₁₀ alkyl, a substituted or unsubstituted aryl, such as phenyl, benzyl, tolyl, o-xylyl, a fluorinated C₁-C₁₀ linear, branched or cyclic hydrocarbon or a halogen. R₂ is preferably methyl, p-toluoyl, trifluoromethyl or fluorine.

As used herein, the term "organic solvent" refers to "a component of a solution which is present in excess, or whose physical state is in the same as that of the solution". Normally the term "organic solvent" includes any substance containing carbon, hydrogen and optionally, oxygen and is normally a liquid at 25 ºC or is easily converted to a liquid by elevating the temperature up to 100 ºC. In addition, the term organic solvent further refers to a combination of two or more of these substances mixed together.

Any solvent having the above described properties can be employed in the present invention. Examples of solvent classes useful herein are, but are not limited to, alcohols, alkoxylated alcohols, aryloxylated alcohols, polyols, glyceryl esters, polymeric ethers, ketones, hydrocarbons and mixtures thereof.

Suitable solvents are benzyl alcohol, benzyl benzoate, 2-benzyloxyethanol, benzyl glycol, 1,2-butanediol, 1,3-butanediol, 1,4-butanediol, 2,3-butanediol, butoxyethyl acetate (regular), butyl acetate, *t*-butyl acetate, n-butanol, *t-*butanol, butylene glycol, butylene glycol proprionate, butyloctanol, butyloctyl benzoate, C7-8 Isoparaffin, C8-9 Isoparaffin, C9-11 Isoparaffin, C9-13 isoparaffin, cyclohexanedimethanol, cyclohexanone, decane, 1,10-decanediol, diethoxydiglycol, dimethyl glutarate, dimethyl maleate, dioxolane, dipropylene glycol, dipropylene glycol dimethyl ether, dipropyl oxalate, ethoxydiglycol, ethoxydiglycol acetate, ethyl acetate, ethylene glycol, ethylene glycol mono-n-butyl ether, ether hexanediol, ethylhexyl acetate, ethylhexyl benzoate, ethyl lactate, glycerine, hexane, hexandiol, 1,2-hexanediol, 1,2,6-hexanetriol, hexylene glycol, isobutoxypropanol, isododecane, isopentyldiol, isopropyl acetate, isopropyl alcohol, 3-methoxybutanol, methoxybutanol, methoxyethanol, methoxyisopropanol, methoxymethylbutanol, methyl acetate, methyl hexyl ether, pentylene glycol, 2-phenoxyethanol, 1-phenoxy-2-propanol, 2-phenyethanol, propanediol, propyl acetate, propyl alcohol, propylene glycol, trimethyl-1,3-pentanediol, acetone, methyl ethyl ketone, methyl tert butyl ether, ethyl acetate, methyl acetate, propyl acetate, isopropyl acetate, butyl acetate, isobutyl acetate, diisopropyl ether toluene, methyl cyclohexane and xylene.

The organic solvents useful herein have preferably a molecular weight of about 200 or less, more preferably about 185 or less, even more preferably about 160 or less.

As used herein, the term "base" refers to a substance that tends to accept a proton. Typical bases are alkaline hydroxide and amines, as for example NaOH, KOH or R-NH₂, including but not limited to DMAP, 4-dimethylaminopyridine, N,N-diisopropylethylamine and triethylamine. An inorganic base is a substance, which contains a metal cation and does not contain an organic moiety, as compared to an organic base, which is a substance that contains an organic moiety. Typical inorganic bases are for example, metal hydroxide, such as sodium hydroxide and potassium hydroxide, metal carbonates, such as sodium carbonate and potassium bicarbonate.

As used herein, the term "acid" refers to a substance that tends to release a proton. The term "acid" contemplates all inorganic or organic acids. Acids preferred for the present invention are mineral acids, such as hydrogen halides and their solutions (hydrochloric acid (HCl), hydrobromic acid (HBr), hydroiodic acid (HI)), halogen oxoacids, such as hypochloric acid, chloric acid, perchloric acid, periodic acid and corresponding compounds for bromine and iodine, sulfuric acid (H₂SO₄), nitric acid (HNO₃), phosphoric acid (H₃PO₄), fluoroboric acid, sulfonic acids, such as methanesulfonic acid (or mesylic acid, CH₃SO₃H), ethanesulfonic acid (or esylic acid, CH₃CH₂SO₃H), benzenesulfonic acid (or besylic acid, C₆H₅SO₃H), p-toluenesulfonic acid (or tosylic acid, CH₃C₆H₄SO₃H), trifluoromethanesulfonic acid (or triflic acid, CF₃SO₃H), carboxylic acids, such as acetic acid, trifluoroacetic, citric acid, formic acid, gluconic acid, lactic acid, oxalic acid, tartaric acid, succinic acid, malic acid

The term "phase transfer catalyst" is used herein to represent any catalyst which can effectively facilitate the transfer of ions or other reactive or functional chemical species or groups across the phase interface.

The term "purification" refers to the process wherein a purified drug substance can be obtained. The term "industrial purification" refers to purifications which can be carried out on an industrial scale such as solvent extraction, filtration, slurring, washing, phase separation, evaporation, centrifugation or crystallisation.

As used herein, the term, "solvent extraction" refers to the process of separating components of a mixture by using a solvent which possesses greater affinity for one component, and may therefore separate said one component from at least a second component which is less miscible than said one component with said solvent.

The term "filtration" refers to the act of removing solid particles greater than a predetermined size from a feed comprising a mixture of solid particles and liquid. The expression "filtrate" refers to the mixture less the solid particles removed by the filtration process. It will be appreciated that this mixture may contain solid particles smaller than the predetermined particle size. The expression "filter cake" refers to residual solid material remaining on a feed side of a filtration element.

As used herein, the term "slurring" refers to any process which employs a solvent to wash or disperse a crude product.

As used herein, the term "washing" refers to the process of purifying a solid mass (e.g., crystals) by passing a liquid over and/or through the solid mass, as to remove soluble matter. The process includes passing a solvent, such as distilled water, over and/or through a precipitate obtained from filtering, decanting, or a combination thereof. For example, in one embodiment of the invention, washing includes contacting solids with solvent or solvent mixture, vigorously stirring (e.g., for two hours), and filtering. The solvent can be water, can be an aqueous solvent system, or can be an organic solvent system. Additionally, the washing can be carried out with the solvent having any suitable temperature. For example, the washing can be carried out with the solvent having a temperature between about 0 ºC and about 100 ºC.

The term "phase separation" refers to a solution or mixture having at least two physically distinct regions.

The term "evaporation" refers to the change in state of solvent from liquid to gas and removal of that gas from the reactor. Generally gas is removed by vacuum applied across the membrane. Various solvents may be evaporated during the synthetic route disclosed herein. As known to those of skill in the art, each solvent may have a different evaporation time and/or temperature.

The term "crystallization" refers to any method known to a person skilled in the art such as crystallization from single solvent or combination of solvents by dissolving the compound optionally at elevated temperature and precipitating the compound by cooling the solution or removing solvent from the solution or both. It further includes methods such as solvent/antisolvent or precipitation.

The term "aryl" refers to an aromatic ring which contains from 3 to 12 carbon atoms, preferably from 6 to 12 carbon atoms. Examples of aryl include phenyl, tropyl, indenyl, naphtyl, azulenyl, biphenyl and antracenyl, and preferably phenyl.

The term "alkyl" comprises linear or branched alkyl groups. Examples of alkyl include methyl, ethyl, n-propyl, iso-propyl, n-butyl, iso- butyl, tert-butyl, sec-butyl, pentyl, iso-pentyl, hexyl, heptyl, octyl, nonyl and decyl, and the isomers thereof.

By the feature "polymorph form X essentially free of polymorph form Y" as used herein means that clevidipine butyrate polymorph form X prepared in accordance with this invention contains less than about 1% of form Y as measured by XRD. By the feature "polymorph form X substantially free of polymorph form Y" as used herein means that the clevidipine butyrate polymorph form X prepared in accordance with this invention contains less than about 15%, preferably less than about 10%, and more preferably less than about 5-8% of form Y as measured by XRD.

### DETAILED DESCRIPTION OF THE INVENTION

The first aspect of the invention relates to a novel method for the preparation of clevidipine butyrate in high purity and yields. The first step of the new improved method is described below.
i)- Reaction of compound I with methyl-3-Amino-crotonate, to obtain clevidipine butyrate in the presence of an organic solvent, preferably at a temperature below 150 ºC. Preferably at a temperature ranging from 40 ºC to 150 ºC and maintained for a sufficient time until the reaction is complete. Most preferably the process is carried out at a temperature ranging from 10 ºC below the refluxing temperature and refluxing temperature, preferably to a temperature ranging from 5 ºC below refluxing temperature and refluxing temperature. Equal molar ratios of compound I and methyl-3-Aminocrotonate are used. No excess of methyl-3-Amino-crotonate is needed. Suitable organic solvents include alcohols such as benzyl alcohol, 2-benzyloxyethanol, benzyl glycol, 1,10-decanediol, hexanediol ,1,2-butanediol, 1,3-butanediol, 1,4-butanediol, 2,3-butanediol, methanol, ethanol, isopropyl alcohol, n-propanol, *tert-*butanol, diethoxydiglycol, ethoxydiglycol, ethylene glycol, ethylene glycol mono-n-butyl ether, hexandiol, 1,2-hexanediol, 1,2,6-hexanetriol, hexylene glycol, isobutoxypropanol, *iso*pentyldiol, 3-methoxybutanol, methoxybutanol, methoxyethanol, methoxyisopropanol, methoxymethylbutanol, pentylene glycol, 2-phenoxyethanol, 1-phenoxy-2-propanol, 2-phenyethanol, propanediol, propylene glycol, trimethyl-1,3-pentanediol; ketones such as acetone, methyl ethyl ketone, ethers such as methyl *tert*-butyl ether; esters such as methyl acetate, ethyl acetate, propyl acetate, isopropyl acetate, butyl acetate, isobutyl acetate; and hydrocarbons selected from toluene, methyl cyclohexane, xylene and mixtures thereof. The preferred solvents are ethanol, isopropyl alcohol and n-propyl alcohol. Most preferably, isopropyl alcohol (IPA). The obtained compound can be purified by standard purification and isolation methods using solvents such as water, organic solvents or mixtures thereof. Suitable organic solvents include ethers, such as diisopropyl ether (DIPE), methyl *tert*-butyl ether, diethyl ether, linear and cyclic hydrocarbons such as toluene, methyl cyclohexane, xylene, n-hexane and short chain alcohols selected from methanol, ethanol, IPA, n-propanol and mixtures thereof. Preferably the organic solvent is methyl cyclohexane.

It has been found that clevidipine butyrate is obtained in a high purity and yield. Equal molar ratios of compound I and methyl-3-Amino-crotonate are preferably used. As a result of that, no excess of methyl-3-Amino-crotonate is needed to be industrially feasible, minimising or eliminating the generation of impurities. The use of methyl-3-Amino-crotonate in the last step prevents the synthesis of impurities very similar in nature to clevidipine butyrate, which would be very difficult to separate and would increase the number of steps to obtain clevidipine butyrate of high purity. The necessary starting materials for the last steps and co-reactants are non-toxic and they are either commercial or may be obtained with less synthetic steps compared with the processes disclosed in the art. The process is not restricted to any kind of solvent, which means that low toxic solvent can be used without losing process efficiency. Moreover, the process of the invention permits the isolation of some of the intermediates, which is a clear advantage, as it is explained below.

The obtained compound, clevidipine butyrate, can be further purified by using standard purification and isolation techniques such as, crystallisation, washing or slurring with organic solvents, water or mixtures thereof. Suitable organic solvents include alcohols such as benzyl alcohol, 2-benzyloxyethanol, benzyl glycol, 1,10-decanediol, hexanediol, 1,2-butanediol, 1,3-butanediol, 1,4-butanediol, 2,3-butanediol, methanol, ethanol, isopropyl alcohol, n-propanol, *tert*-butanol, diethoxydiglycol, ethoxydiglycol, ethylene glycol, ethylene glycol mono-n-butyl ether, hexandiol, 1,2-hexanediol, 1,2,6-hexanetriol, hexylene glycol, *iso*butoxypropanol, *iso*pentyldiol, 3-methoxybutanol, methoxybutanol, methoxyethanol, methoxy*iso*propanol, methoxymethylbutanol, pentylene glycol, 2-phenoxyethanol, 1-phenoxy-2-propanol, 2-phenyethanol, propanediol, propylene glycol, trimethyl-1,3-pentanediol, ethers such as THF and diisopropylether (DIPE) and ketones such as, acetone, methyl ethyl ketone (MEK) and methyl isobutyl ketone (MIBK) and mixtures thereof. Preferably purification is carried out using water, isopropanol, propanol, acetone, methanol, DIPE and mixtures thereof.

Disclosed are compound II, 2-(2',3'-Dichloro-benzylidene)-3-oxo-butyric acid and salts thereof as well as their use as intermediates for the preparation of clevidipine butyrate. Salts of benzylidine acid include salts of alkaline metal cations such as potassium and sodium salts. These intermediate compounds are obtained in high purity and yield and can be easily isolated and purified by using standard industrial methods, avoiding the use of column chromatography.

A further aspect of the invention is to provide a method to prepare compound II or a salt thereof, comprising the saponification of compound (III), wherein compound (III) is preferably obtained by the reaction of compound (IV) and (V), as depicted in the scheme below, 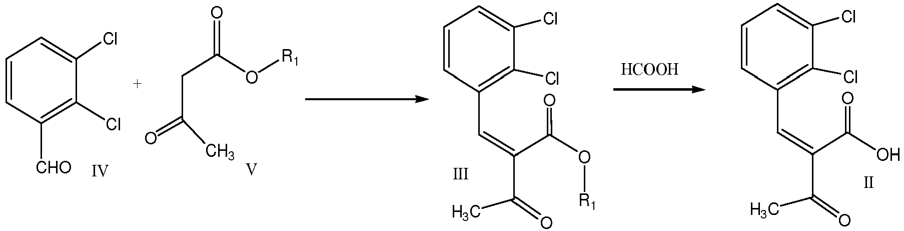 which preferably comprises the steps of:
Reacting compound IV (2,3-Dichloro-benzaldehyde with a compound of formula V, CH₃-CO-CH₂-COOR₁, wherein R₁ is selected from an alkyl, preferably a methyl, ethyl, propyl, isopropyl, butyl, *tert*-butyl, or from an aryl, preferably a phenyl, benzyl and trityl, in the presence of a base and an acid to obtain compound III, wherein R₁ is as defined above. Suitable acids are selected from acetic acid, formic acid, trifluoroacetic acid, sulphuric acid, hydrochloric acid, hydrobromic acid and mixtures thereof. Suitable organic bases are selected from secondary and tertiary linear or cyclic amines, such as piperidine, morpholine, triethyl amine, diisopropyl ethyl amine, and diethyl amine. Preferably bases are selected from piperidine and morpholine. Salts of these bases such as morpholine acetate can also be used.

Followed by hydrolysis of compound III, wherein R₁ is selected from an alkyl, preferably a methyl, ethyl, propyl, isopropyl, butyl, *tert*-butyl, or from an aryl, preferably a phenyl, benzyl and trityl, in the presence of an acid, to obtain compound II or a salt thereof. The hydrolysis of compound III is carried out in the presence of organic or inorganic acids. Suitable organic acids are carboxylic acids, such as formic acid, acetic acid, trifluoroacetic acid, oxalic acid, benzoic acid and mixtures thereof. Suitable inorganic acids can be selected from HCl, HBr, H₂SO₄ and mixtures thereof. The most preferred acids are formic acid and sulphuric acid. The obtained compound II can be purified by means of standard industrial purification and isolation methods. The obtained compound II can be purified by means of standard industrial purification and isolation methods. Preferably compound II is purified by washing with an organic solvent, in which the generated acid has low solubility such as toluene, hexane, methyl *tert*-butyl ether, methyl cyclohexane, xylene and mixtures thereof. The most preferred solvent is toluene. Compound II can be obtained as cis or trans enantiomer and mixtures thereof. The ratio of each enantiomer in the mixture is irrelevant to for their use as reactants in the next step (step b or condensation of compound II).

Optionally an alkali metal base may be added over compound II, followed by stirring for a period of time sufficient to form the alkali metal salt of compound II. Salts of benzylidine acid include salts of alkaline metal cations such as potassium, sodium and lithium.

Optionally, compound II or salt thereof may be purified by standard industrial purification methods.

The second step of the process of the invention relates to the preparation of compound I, (2,3-Dichloro-benzylidene)-3-oxo-butyric acid butyryloxymethyl ester and disclosed is their use as intermediates for the preparation of clevidipine butyrate. See reaction scheme below.

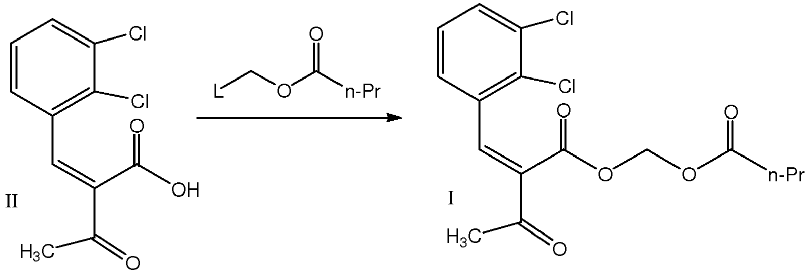

A method for the preparation of compound I comprises:
Condensation of compound II or a salt thereof with a compound of formula L-CH₂OCO-n-Pr, wherein L is a leaving group to obtain Compound I (2-(2',3'-Dichloro-benzylidene)-3-oxo-butyric acid butyryloxymethyl ester). The term "leaving group" includes to halogens and sulphonyloxy groups, represented by -OSO₂R₂ wherein the preferred R₂ is p-toluoyl, trifluoromethyl, fluorine or alkyl, preferably methyl. Most preferably the leaving group is Cl. The condensation reaction is carried out in the presence of a base and an organic solvent. The base can be organic or inorganic. Suitable organic bases are selected from secondary and tertiary amines, such as triethyl amine, diisopropyl ethyl amine, and diethyl amine. Suitable inorganic bases are selected from, ammonium hydroxide aqueous or alcoholic solution, sodium bicarbonate, sodium carbonate, potassium carbonate and potassium bicarbonate. Preferably bases are selected from triethyl amine and diisopropyl ethyl amine.

Suitable organic solvent for the condensation step are selected from at least benzyl alcohol, 2-benzyloxyethanol, benzyl glycol, 1,10-decanediol, hexanediol ,1,2-butanediol, 1,3-butanediol, 1,4-butanediol, 2,3-butanediol, methanol, ethanol, isopropyl alcohol, n-propanol, *tert*-butanol, diethoxydiglycol, ethoxydiglycol, ethylene glycol, ethylene glycol mono-n-butyl ether, hexandiol, 1,2-hexanediol, 1,2,6-hexanetriol, hexylene glycol, isobutoxypropanol, isopentyldiol, 3-methoxybutanol, methoxybutanol, methoxyethanol methoxyisopropanol, methoxymethylbutanol, pentylene glycol, 2-phenoxyethanol, 1-phenoxy-2-propanol, 2-phenyethanol, propanediol, propylene glycol, trimethyl-1,3-pentanediol, acetone, methyl ethyl ketone, methyl tert butyl ether, ethyl acetate, methyl acetate, propyl acetate, isopropyl acetate, butyl acetate, isobutyl acetate, diisopropyl ether, dimethylacetamide, cyclic hydrocarbones such as toluene, xylene, methyl cyclohexane, dichloromethane and mixtures thereof. Preferred organic solvents include; dichloromethane, acetone, methylethyl ketone, methyl isobutyl ketone, dimethylacetamide, toluene, xylene, methyl cyclohexane, methyl *tert*-butyl ether, THF, methyl acetate, ethyl acetate, propyl acetate, isopropyl acetate, butyl acetate, isobutyl acetate, and mixtures thereof. The most preferred organic solvent is ethanol, isopropyl alcohol and n-propyl alcohol, diisopropyl ether or mixtures thereof

As previously mentioned, Compound II can be obtained as cis or trans enantiomer and mixtures thereof. The ratio of each enantiomer in the mixture is irrelevant to for its use of compound II as a reactant in this step.

Optionally, the condensation reaction can be carried out in presence of phase transfer catalysts such as tetrabutyl ammonium bromide, tetrabutyl ammonium chloride, tetrabutyl ammonium sulphate and polyethylene glycols.

Optionally, compound I may be purified by standard industrial methods.

These intermediate compounds, compounds II and I, are obtained in high purity and can be easily isolated and purified, as solids, by using standard industrial methods, avoiding the use of column chromatography which is a clear advantage.

Disclosed are also new crystalline forms of clevidipine butyrate, useful to improve the pharmaceutical performance of pharmaceutical products, and methods for preparing thereof. There is a need in the art form new polymorphic forms of clevidipine butyrate and for their preparation as pure forms. The preparation methods herein described, allow the obtainment of polymorphs of clevidipine butyrate free of other polymorphic forms. Mixtures of polymorphic forms were also obtained as described in some of the experiments.

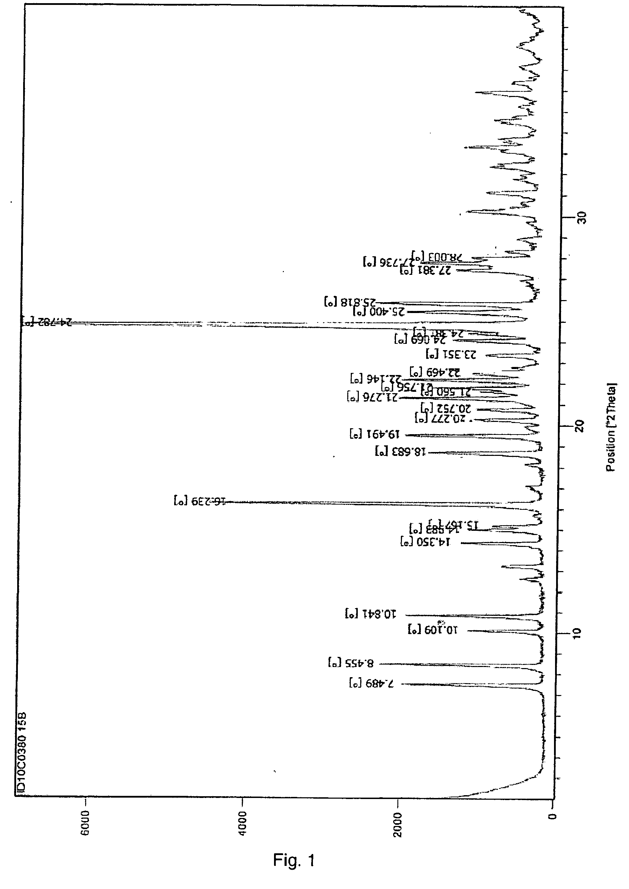

In one embodiment, crystalline from A of clevidipine butyrate is disclosed. Form A of clevidipine butyrate has a m.p. (melting point) 138.8 ºC (measured by DSC). Melting point was measure using a Jade DSC by Perkin Elmer. Form A of clevidipine butyrate is also characterised by substantially the same XRD pattern as that depict in figure 1. The diffractogram was obtained by using a XPERT PRO by PANalytical

The XRD pattern for each crystalline form is unique, exhibiting an unique set of diffraction peaks, which can be expressed in 2 theta angles (º), d-spacings (Å) and/or relative peak intensities. Large variations of relative peak intensities may be observed due to preferred orientation resulting from difference in crystal morphologies. Identification of the exact crystal form of a compound should be based primarily on the observed 2 theta angles or d-spacings with lesser importance focused on relative intensities. Therefore, Form A of clevidipine butyrate is also characterised by and XRD pattern expressed in terms of 2 theta angle, wherein the XRD pattern comprises 2 theta angles at four or more positions, selected from the group consisting of: 8.5 ±0.2, 16.2 ±0.2, 18.7 ±0.2, 22.2 ±0.2, 24.8 ±0.2, 25.4 ±0.2, 25.8 ±0.2, 27.7 ±0.2.

Although, one skilled in the art can identify form A of clevidipine butyrate from these peaks, in some circumstances it may be desirable to relay upon additional 2 theta angles for the identification of form A of clevidipine butyrate. Form A of clevidipine butyrate exhibits 2 theta angle peaks, in addition to the foregoing peaks, at essentially the following positions: 10.84 ±0.2, 14.35 ±0.2, 19.49 ±0.2, 21.2 ±0.2, 21.76 ±0.2, 24.07 ±0.2, 27.38 ±0.2, 28.0 ±0.2.

Form A of clevidipine butyrate is also by substantially the same XRD pattern as that depict in figure 1.

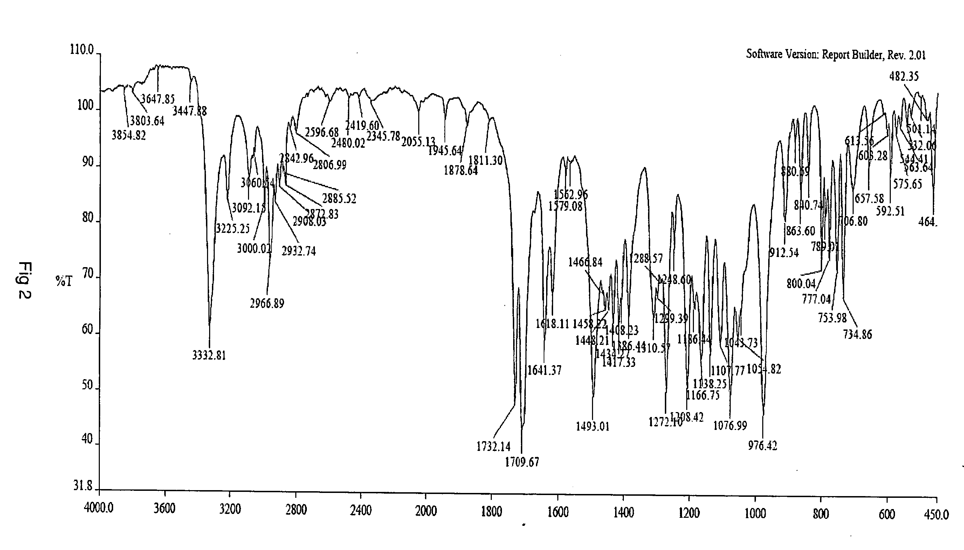

Form A of clevidipine butyrate is also by substantially the same IR pattern as that depict in figure 2. The FTIR spectrum was obtained using a Spectrum 100 by Perkin Elmer

A process for the preparation of polymorph A comprises:
i) Dissolving clevidipine butyrate in an organic solvent at temp above 65 ºC;
ii) Optionally, adding activated charcoal at room temperature for half and hour and removing the charcoal from the media;
iii) Adding water to the clevidipine solution at room temperature and stirred the mixture until a precipitate is obtained;
iv) Removing clevidipine from the medium.

The process herein described for the preparation of polymorph A allows the obtainment of the polymorphic form A essentially free of other polymorphic forms.

In another embodiment, crystalline from B of clevidipine butyrate is disclosed. Form B of clevidipine butyrate has a m.p. 142.8 ºC (measured by DSC). Melting point was measure using a Jade DSC by Perkin Elmer. Form B of clevidipine butyrate is also characterised by substantially the same XRD pattern as that depicted in figure 9. Form B of clevidipine butyrate is also characterised by XRD pattern expressed in terms of 2 theta angle, wherein the XRD pattern comprises 2 theta angles at four or more positions selected from the group consisting of XRD: 7.24 ±0.2, 8.95 ±0.2, 10.29 ±0.2, 17.02 ±0.2, 20.21 ±0.2, 21.87 ±0.2, 22.57 ±0.2, 22.87 ±0.2, 24.23 ±0.2, 25.39 ±0.2.

Form B of clevidipine butyrate exhibits 2 theta angle peaks in addition to the foregoing peaks at essentially the following positions: 13.22 ±0.2, 13.39 ±0.2, 16.19 ±0.2, 23.12 ±0.2, 24.66 ±0.2.

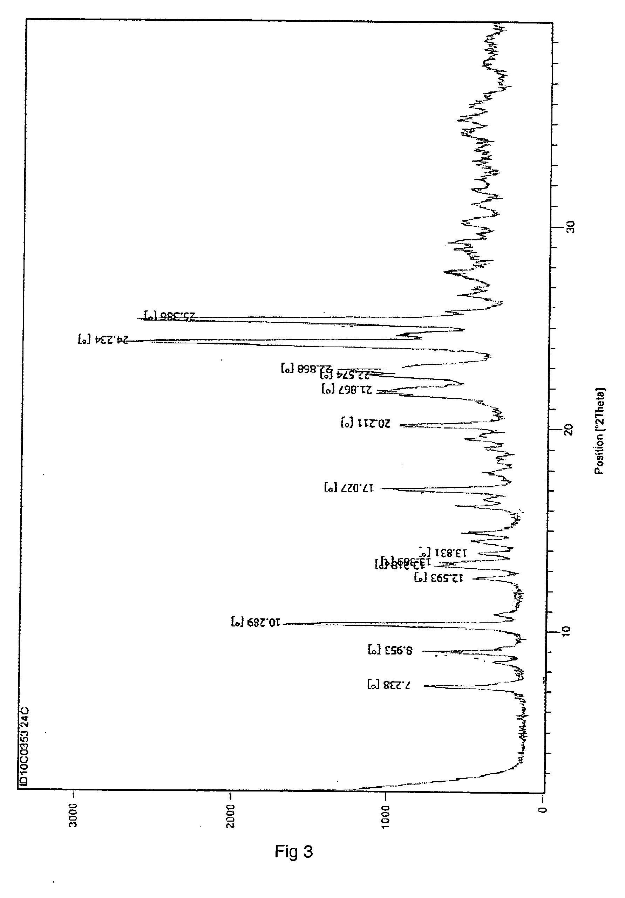

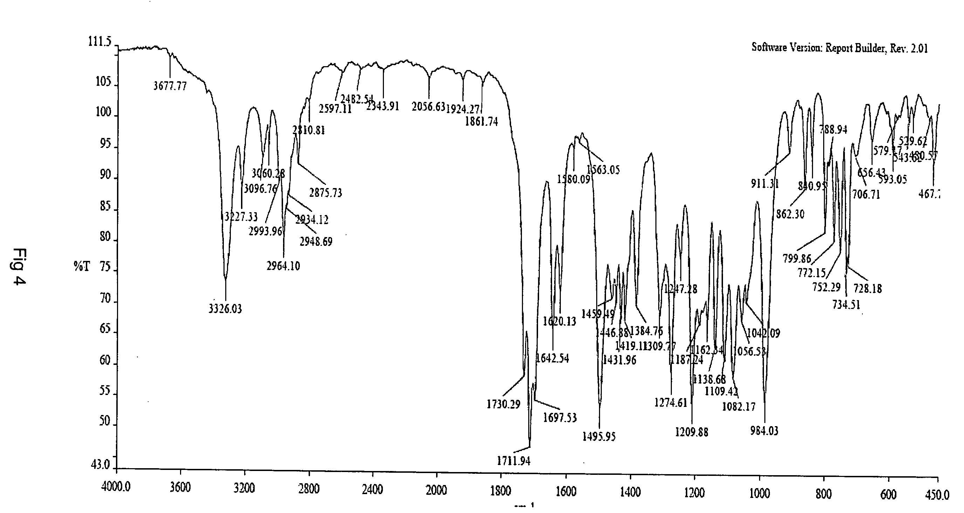

Form B of clevidipine butyrate is also by substantially the same XRD patent as that depict in figure 3. The diffractogram was obtained by XPERTO PRO by PANalytical. Form B of clevidipine butyrate is also by substantially the same IR pattern as that depict in figure 4. The FTIR spectrum was obtained using a Spectrum 100 by Perkin Elmer.

A process for the preparation of polymorph B comprises:
i) Dissolving clevidipine butyrate in an organic solvent;
ii) Adding water at room temperature and stirring the mixture until a precipitate is obtained;
iii) Removing clevidipine from the medium.

The process herein described for the preparation of polymorph B allows the obtainment of the polymorphic form B essentially free of other polymorphic forms.

Furthermore, the disclosure relates to a pharmaceutical composition of clevidipine butyrate comprising any one of the polymorphs described herein and their use for the reduction of blood pressure when oral therapy is not feasible or not desirable.

In addition, the disclosure relates to a pharmaceutical composition of clevidipine butyrate prepared by any one of the methods described herein and their use for the reduction of blood pressure when oral therapy is not feasible or not desirable.

Moreover, the disclosure also relates to the use of these novel compounds, II and III, for the preparation of clevidipine butyrate.

The inventors have developed formulations comprising polymorph I and/or II of clevidipine butyrate with excellent properties as pharmaceutical products.

The aspects (clauses 1, 30-32, 38, 42, 56 and 59) and the preferred embodiments (clauses 2-29, 33-37, 39-41, 43-55, 57-58 and 60) of the present disclosure are described in the following clauses below and the scope of the present invention is defined solely by the claims.
Clause 1.- A process for the preparation of clevidipine butyrate comprising the step:
   (i) reacting a compound of formula (I): 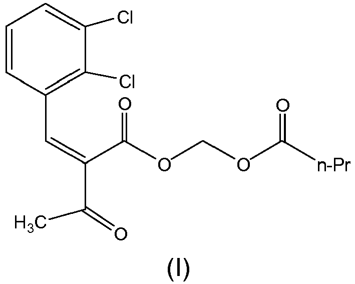
   with methyl 3-amino crotonate to obtain clevidipine butyrate, wherein the step (i) is carried out in the presence of at least an organic solvent or a solvent mixture comprising an organic solvent.
Clause 2.- The process according to clause 1, wherein the ratio of methyl 3-aminocrotonate to compound of formula (I) ranges from 2:1 to 1:2, preferably from 1.25:1 to 1:1.25, and more preferably is about 1:1.
Clause 3.- The process according to any one of the preceding clauses, wherein the step (i) is carried out at a temperature below 150 ºC.
Clause 4.- The process according to the preceding clause, wherein the step (i) is carried out at a temperature ranging from 50 ºC to 120 ºC.
Clause 5.- The process according to any one of the preceding clauses, comprising at least one organic solvent selected from benzyl alcohol, 2-benzyloxyethanol, benzyl glycol, 1,10-decanediol, hexanediol, 1,2-butanediol, 1,3-butanediol, 1,4-butanediol, 2,3-butanediol, methanol, ethanol, isopropyl alcohol, n-propanol, *tert*-butanol, diethoxydiglycol, ethoxydiglycol, ethylene glycol, ethylene glycol mono-n-butyl ether, hexandiol, 1,2-hexanediol, 1,2,6-hexanetriol, hexylene glycol, *iso*butoxypropanol, *iso*pentyldiol, 3-methoxybutanol, methoxybutanol, methoxyethanol, methoxyisopropanol, methoxymethylbutanol, pentylene glycol, 2-phenoxyethanol, 1-phenoxy-2-propanol, 2-phenyethanol, propanediol, propylene glycol, trimethyl-1,3-pentanediol, acetone, methyl ethyl ketone, methyl tert butyl ether, ethyl acetate, methyl acetate, propyl acetate, isopropyl acetate, butyl acetate, isobutyl acetate, diisopropyl ether toluene, methyl cyclohexane, xylene and mixtures thereof.
Clause 6.- The process according to any one of the preceding clauses, wherein the preferred organic solvent is selected form methanol, ethanol, isopropyl alcohol, n-propanol, *tert*-butanol, acetone, methyl ethyl ketone, methyl tert butyl ether, ethyl acetate, methyl acetate, propyl acetate, isopropyl acetate, butyl acetate, isobutyl acetate, diisopropyl ether toluene, methyl cyclohexane, xylene and mixtures thereof.
Clause 7.- The process according to the preceding clause, wherein the most preferred organic solvent is ethanol, isopropyl alcohol and n-propyl alcohol, diisopropyl ether or mixtures thereof.
Clause 8.- The process according to any one of the preceding clauses, wherein the compound of formula (I) is prepared by esterification of a compound of formula (II) or a salt thereof: 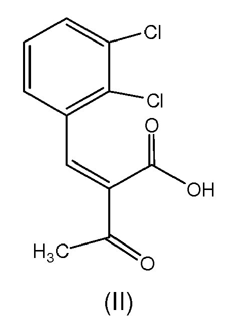 with a compound of formula L-CH₂OCO-n-Pr, wherein L is a leaving group.
Clause 9.- The process according to preceding clause, wherein the esterification is carried out in the presence of at least one organic and/or inorganic base.
Clause 10.- The process according to the preceding clause, wherein the base is selected from secondary and tertiary amines, ammonium hydroxide solution, carbonates, bicarbonates of alkali, alkali earth metals and any mixtures thereof.
Clause 11.- The process according to the preceding clause, wherein the preferred base is triethyl amine, diisopropyl ethyl amine, ammonium hydroxide solution, sodium bicarbonate, sodium carbonate, potassium carbonate, potassium bicarbonate and any mixtures thereof.
Clause 12.- The process according to any one of the clauses 8 to 11, wherein the leaving group is a carboxyl ester, an halogen or a sulphonyloxy groups represented by -OSO₂R₂, , wherein the preferred R₂ is alkyl, p-toluoyl, trifluoromethyl or fluorine.
Clause 13.- The process according to the preceding clause, wherein the leaving group is trifluoromethylsulfonyl, para-nitrobenzenesulfonyl, para-toluenesulfonyl, methylsulfonyl, trifluoroacetyl, para-nitrobenzoyl, para-methylbenzoyl, acetyl, iodine, bromine, chlorine or fluorine.
Clause 14.- The process according to the preceding clause, wherein the leaving group is chlorine.
Clause 15.- The process according to any one of the clauses 8 to 14, wherein the esterification step is carried out in the presence of an organic solvent, preferably the organic solvent is selected from: dichloromethane; ketones such as acetone, methylethyl ketone, methyl isobutyl ketone, dimethylacetamide; cyclic hydrocarbones such as toluene, xylene, methyl cyclohexane; linear and cyclic ethers such as methyl *tert*-butyl ether, THF and esters such as methyl acetate, ethyl acetate propyl acetate, isopropyl acetate, butyl acetate, isobutyl acetate; and mixtures thereof.
Clause 16.- The process according to preceding clause, wherein the esterification is carried out in presence of phase transfer catalysts, preferably tetrabutyl ammonium bromide, tetrabutyl ammonium chloride, tetrabutyl ammonium sulphate, polyethylene glycol and mixtures thereof.
Clause 17.- The process according to any one of the clauses 8 to 16, wherein the compound of formula (II) is obtained by saponification of the compound of formula (III), wherein R₁ is selected from: an alkyl, preferably selected from methyl, ethyl, propyl, isopropyl, butyl and *tert*-butyl; and an aryl, preferably selected from trityl, phenyl and benzyl. 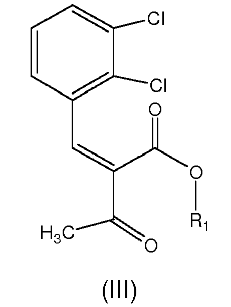
Clause 18- The process according to the preceding clause, wherein the saponification is carried out in the presence of an acid or a base.
Clause 19.- The process according to the preceding clause, wherein the saponification is carried out in the presence of an acid.
Clause 20.- The process according to the preceding clause, comprising at least one acid selected from: hydrogen halides and their solutions, hydrochloric acid (HCl), hydrobromic acid (HBr), hydroiodic acid (HI); halogen oxoacids, such as hypochloric acid, chloric acid, perchloric acid, periodic acid and corresponding compounds for bromine and iodine; sulfuric acid (H₂SO₄); nitric acid (HNO₃); phosphoric acid (H₃PO₄); fluoroboric acid; sulfonic acids, such as methanesulfonic acid (or mesylic acid, CH₃SO₃H), ethanesulfonic acid (or esylic acid, CH₃CH₂SO₃H), benzenesulfonic acid (or besylic acid, C₆H₅SO₃H), p-toluenesulfonic acid (or tosylic acid, CH₃C₆H₄SO₃H), trifluoromethanesulfonic acid (or triflic acid, CF₃SO₃H); carboxylic acids, such as acetic acid, trifluoroacetic, citric acid, formic acid, gluconic acid, lactic acid, oxalic acid, tartaric acid, succinic acid, malic acid; and mixtures thereof.
Clause 21.- The process according to the preceding clause, comprising at least one acid selected from trifluoroacetic acid, sulphuric acid, hydrochloric acid, hydrobromic acid, formic acid, trifluoroacetic acid and mixtures thereof.
Clause 22.- The process according to the preceding clause, comprising at least one acid selected from formic acid, trifluoroacetic acid and mixtures thereof.
Clause 23.- The process according to the clause 20, comprising at least one acid selected from sulphuric acid, hydrochloric acid, hydrobromic acid and mixtures thereof.
Clause 24.- The process according to any one of the clauses 17 to 23, wherein the compound of formula (III) is obtained by reacting compound of formula (IV) (2,3-dichloro-benzaldehyde) with a compound of formula (V) (CH₃-CO-CH₂-COOR₁): 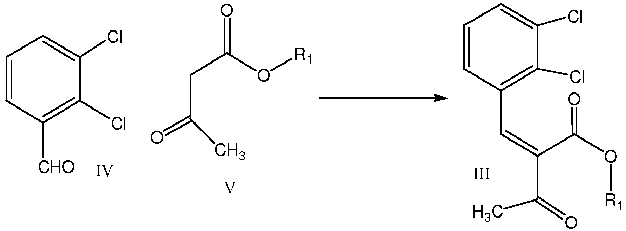 wherein R₁ is as defined above in the Clause 17.
Clause 25.- The process according to the preceding clauses, wherein the reaction between the compound of formula (IV) and the compound of formula (V) takes place by adding of a base and/or an acid.
Clause 26.- The process according to the preceding clauses, comprising an acid selected from acetic acid, formic acid, trifluoroacetic acid, sulphuric acid, hydrochloric acid, hydrobromic acid and mixtures thereof and a base selected from secondary and tertiary linear or cyclic amines, such as piperidine, morpholine, triethyl amine, diisopropyl ethyl amine, and diethyl amine. Preferably bases are selected from piperidine and morpholine. Salts of these bases such as morpholine acetate can also be used.
Clause 27.- The process according to any one of the preceding clauses, wherein the clevidipine butyrate obtained in the step (i) is purified.
Clause 28.- The process according to the preceding clause, wherein the clevidipine butyrate is at least purified by a method selected from solvent extraction, filtration, slurring, washing, phase separation, evaporation, centrifugation, crystallisation or any combination thereof.
Clause 29.- The process according to the preceding clause, wherein the clevidipine butyrate is at least purified by a method selected from slurring, washing, crystallisation or any combination thereof.
Clause 30.- A compound of formula (I): 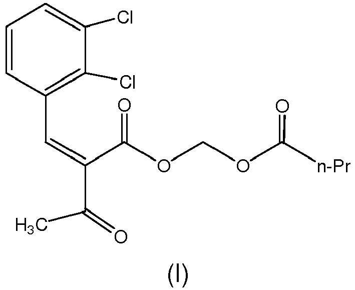
Clause 31.- A compound of formula (II) or a salt thereof: 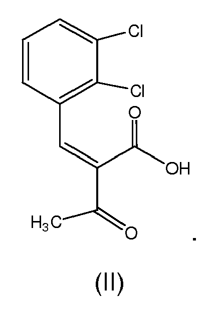
Clause 32.- A crystalline polymorph clevidipine butyrate form A that exhibits an X-ray powder diffraction pattern having characteristic peaks expressed in degrees 2-theta at approximately 8.5 ±0.2, 16.2 ±0.2, 18.7 ±0.2, 22.1 ±0.2, 24.8 ±0.2, 25.4 ±0.2, 25.8 ±0.2, 27.7 ±0.2.
Clause 33.- The crystalline polymorph according to preceding clause which is substantially free of the B polymorph.
Clause 34.- The crystalline polymorph according to the preceding clauses which is essentially free of the clevidipine butyrate B polymorph.
Clause 35.- The crystalline polymorph according to any one of the clauses 32 to 34, characterized by the X-ray powder diffraction pattern shown in Figure 1.
Clause 36.- The crystalline polymorph according to any one of the clauses 32 to 35 which is characterized by an IR absorption spectrum having characteristic peaks expressed in cm⁻¹ at approximately 3333 ±1, 3225 ±1, 2967 ±1, 1732 ±1, 1710 ±1, 1641 ±1, 1493 ±1, 1272 ±1, 1208 ±1, 1138 ±1, 1077 ±1, 976 ±1, 735 ±1, 464 ±1.
Clause 37.- The crystalline polymorph of clause 36, characterized by the IR absorption spectrum shown in Figure 2.
Clause 38.- A pharmaceutical composition comprising any one of the polymorphs as defined in the clauses 32 to 37.
Clause 39.- The pharmaceutical composition according to the preceding clause in the form of emulsion for injection.
Clause 40.- The pharmaceutical composition according to the preceding clause in the form of lipid emulsion.
Clause 41.- The pharmaceutical composition according to any of the clauses 38 to 40, wherein the concentration of clevidipine butyrate is about 0.5 mg/mL.
Clause 42.- A crystalline polymorph clevidipine butyrate form B that exhibits an X-ray powder diffraction pattern having characteristic peaks expressed in degrees 2-theta at approximately 7.2 ±0.2, 9.0 ±0.2, 10.3 ±0.2, 17.0 ±0.2, 20.2 ±0.2, 21.9 ±0.2, 22.6 ±0.2, 22.9 ±0.2, 24.2 ±0.2, 25.4 ±0.2.
Clause 43.- The crystalline polymorph according to preceding clause which is substantially free of the A polymorph.
Clause 44.- The crystalline polymorph according to the preceding clauses which is essentially free of the clevidipine butyrate A polymorph.
Clause 45.- The crystalline polymorph according to any one of the clauses 42 to 44, characterized by the X-ray powder diffraction pattern shown in Figure 3.
Clause 46.- The crystalline polymorph according to any one of the clauses 42 to 45 which is characterized by an IR absorption spectrum having characteristic peaks expressed in cm⁻¹ at approximately 3330 ±1, 3097 ±1, 2965 ±1, 1731 ±1, 1712 ±1, 1697 ±1, 1643 ±1, 1620 ±1, 1419 ±1, 1496 ±1, 1432 ±1, 1274 ±1, 1209 ±1, 1109 ±1, 1082 ±1, 984 ±1, 734.5 ±1, 728 ±1, 468 ±1.
Clause 47.- The crystalline polymorph of clause 46, characterized by the IR absorption spectrum shown in Figure 4.
Clause 48.- A pharmaceutical composition comprising any one of the polymorphs as defined in the clauses 32 to 37 or 42 to 47.
Clause 49.- The pharmaceutical composition according to the preceding clause in the form of emulsion for injection.
Clause 50.- The pharmaceutical composition according to the preceding clause in the form of lipid emulsion.
Clause 51.- The pharmaceutical composition according to any of the clauses 48 to 50, wherein the concentration of clevidipine butyrate is about 0.5 mg/mL.
Clause 52.- The pharmaceutical composition according to the preceding clauses comprising soybean oil, preferably in an amount raging from 150 to 250 mg/mL.
Clause 53.- The pharmaceutical composition according to any of the clauses 51 to 52, comprising glycerine, preferably in an amount raging from 17 to 27 mg/mL.
Clause 54.- The pharmaceutical composition according to any of the clauses 51 to 53, comprising phospholipids, preferably purified egg yolk phospholipids, and also preferably in an amount raging from 17 to 27 mg/mL.
Clause 55.- The pharmaceutical composition according to any of the claims 51 to 54, wherein the composition has an pH ranging from 5.9 to 8.1.
Clause 56.- A process for the preparation of polymorph A according to clauses 32 to 37, which comprises:
   i) Dissolving clevidipine butyrate in an organic solvent at temp above 65 ºC;
   ii) Optionally, adding activated charcoal at room temperature for half and hour and removing the charcoal from the media;
   iii) Adding water to the clevidipine solution at room temperature and stirred the mixture until a precipitate is obtained;
   iv) Removing clevidipine from the medium.
Clause 57.- The process for the preparation of polymorph A according to clause 56, wherein the organic solvents is acetone, diethyl ketone, isopropanol and toluene.
Clause 58.- The process for the preparation of polymorph A according any of the clauses 56 or 57 in which the dissolution step (i) takes place between room temperature and 120 ºC.
Clause 59.- A process for the preparation of polymorph B according to clause 42 to 47, which comprises:
   i) Dissolving clevidipine butyrate in an organic solvent;
   ii) Adding water at room temperature and stirring the mixture until a precipitate is obtained;
   iii) Removing clevidipine from the medium.
Clause 60.- The process for the preparation of polymorph B according to clause 59 wherein the organic solvents is diisopropyl ether and an aqueous solution of methanol.

### EXAMPLES

### Example 1

### Preparation of benzylidine tert-butyl ester (compound III)

50 g (0.285 mol) of 2,3-dichlorobenzaldehyde, 54.17 g (0.342 mol) tert-butyl 3-oxobutanoate, 1.45 g (0.017 mol) of piperidine, 200 ml isopropanol and 1.02 g (0.017 mol) acetic acid were charged in a one litre flask fitted with a reflux condenser. The reaction mass was heated up to 50 ºC and stirred for 1 hour. Afterwards, the mixture was cooled down to 25-30 ºC and stirred for 15 hours. The mixture was further cooled to 10 -15 ºC and stirred for 2 hours. The reaction mass was filtered and washed with 25 ml of chilled isopropanol and 50 ml of hexane. The white coloured solid was dried under reduced pressure at 50-55 °C for 7-8 hours. Yield: 70 g, Molar yield: 77.78 %, HPLC purity: 98-99% and m.p.: 94 to 96 ºC.

### Example 2

### Preparation of benzylidine acid (2-acetyl-3-(2',3'-dichlorophenyl) prop-2-enoic acid) (compound II)

70 g (0.222 mol) of benzylidine tert- butyl ester and 420 ml of formic acid (98-100%) were charged in a one litre flask fitted with reflux condenser and heated up to 50 ºC. The reaction mass was stirred for half an hour, followed by cooling and stirring at 25-30 ºC for 7-8 hours. Formic acid was removed by distillation under reduced pressure at 45 -50 ºC. Afterwards, 140 ml of toluene were charged and distilled out under vacuum at 50 ºC. Additional 140 ml of toluene were charged and stirred at 25-30 ºC for 2 hours. The reaction mass was filtered and the precipitate dried at 50-55 ºC for 8-10 hours. Yield: 48 g, Molar yield: 83.4%, White coloured solid, HPLC purity: 99.68% (Mixture of cis and trans isomers are present in a ratio of 61.79 and 38.02% respectively).
IR: 2931.2, 2625.58, 2540.8, 1733.6, 1623.2, 1675, 1450, 1411.3, 1363.4, 1285.7, 1274.5, 1235.5, 1182.8, 1166, 1049, 917, 898, 821, 796, 715, 656, 601, 567, 542.6, 459.7.

### Example 3

### Preparation of benzylidine acid (2-acetyl-3-(2',3'-dichlorophenyl) prop-2-enoic acid) (compound II)

50 g (0.158 mol) of benzylidine tert- butyl ester and 173 ml of sulphuric acid (50% v/v) were charged in a 0.25 litre flask and stirred for 7-8 hours at 25-30 ºC. The reaction mass was filtered and washed with 100 ml water. The obtained wet cake (35 gm) was stirred in 100 ml toluene at 25 -30 ºC for 1 hour. The solid was filtered and the product dried at 50-55 ºC for 8-10 hours. A white solid obtained. Yield: 32.8 g, Molar yield: 80.15%, HPLC purity: 98.85% (Mixture of cis and trans isomers in the ratio of 39.93 and 60.06% respectively).

### Example 4

### Preparation of Benzylidine butyrate (2-(2',3'-Dichloro-benzylidene)-3-oxo-butyric acid butyryloxymethyl ester) (compound I)

45 g (0.173 mol) of benzylidine acid, 28.95 g (0.286 mol) of triethyl amine and 450 ml dichloromethane were charged in a one litre flask fitted with a reflux condenser. Afterwards, 35.5 g (0.260 mol) of chloromethyl butyrate were slowly added at 25-30 ºC under nitrogen atmosphere. The reaction mass stirred while heated at 40-45 ºC for 30-35 hours. The obtained mixture was cooled down to 30 ºC and washed twice with 200 ml of demineralised water (DM). The organic layer was separated and the dichloromethane distilled out under reduced pressure at 40-50 ºC to obtain a yellowish coloured liquid.
Yield: 64 g, Molar yield: 103 %, HPLC purity: 92.74 % (Mixture of cis and trans isomers are present in a ratio of 62.90 and 37.09 % respectively).
IR: 2968.2, 2936.2, 28877.3, 1761.6, 1703.2, 1678.2, 1625.5, 1558.8, 1452, 1412, 1377, 1363, 1273, 1237.7, 1183, 1158, 1140.4, 1105, 1050, 999, 980, 787, 717, 473, 457.

### Example 5

### Preparation of potassium salt of Benzylidine acid

2.54 g (0.038 mol) of 85% potassium hydroxide and 150 ml *iso*propanol were charged in a 250 ml flask. The mixture was stirred at 50-55 ºC for 30 minutes followed by cooling, down to 25 ºC. 10 g (0.038 mol) of benzylidine acid (compound II) were added into the flask and the mixture stirred at 20-25 ºC for 1 hour. The formed solid was filtered and washed with 10 ml of isopropanol. The wet cake was dried under reduced pressure at 50-55 ºC yielding 8.5 g of a white solid. Molar yield: 75.33 %, HPLC purity: 99.7% (Mixture of cis and trans isomers in the ratio of 85.86 and 14.14 % respectively).

### Example 6

### Preparation of sodium salt of Benzylidine acid

1.54 g (0.038 mol) of sodium hydroxide and 150 ml isopropanol were charged in a 250 ml flask. The mixture was stirred at 50-55 ºC for 30 minutes followed by cooling, down to 25 °C. 10 g (0.038 mol) of benzylidine acid (compound II) were added into the flask and the mixture stirred at 20-25 ºC for 1 hour. The formed solid was filtered and washed with 10 ml of isopropanol. The wet cake was dried under reduced pressure at 50-55 ºC yielding 8.8 g of a white solid. Molar yield: 82.4 %, HPLC purity: 99.8 % (Mixture of cis and trans isomers in the ratio of 97.9 and 2.1 % respectively).

### Example 7

### Preparation of Benzylidine butyrate (2-(2',3'-Dichloro-benzylidene)-3-oxo-butyric acid butyryloxymethyl ester) (compound I)

5.0 g (0.0193 mol) of benzylidine acid, 2.92 g (0.0289 mol) of triethyl amine and 50 ml acetone were charged in a 250 ml flask fitted with a reflux condenser. 3.55 g (0.026mol) of chloromethyl butyrate were slowly added at 25-30 ºC under nitrogen atmosphere. The reaction mass was stirred at 55-58 ºC for 15-20 hours, followed by cooling, down to 30 ºC, and filtering of the inorganic salts. Acetone was distilled out under reduce pressure at 45-50 ºC. The obtained mixture was diluted with 100 ml of ethyl acetate and washed with 100 ml of DM water twice. The organic layer was separated and the ethyl acetate distilled out under reduced pressure at 45-50 ºC to yield a yellowish coloured liquid.
Yield: 6.5 g, Molar yield: 93.8 %, HPLC purity: 91.58 % (Mixture of cis and trans isomers are present in ratio of 64.91 and 35.09 % respectively).

### Example 8

### Preparation of Benzylidine butyrate (2-(2', 3'-Dichloro-benzylidene)-3-oxo-butyric acid butyryloxymethyl ester) (compound I)

2.0 g (0.0067 mol) of potassium salt of benzylidine acid, 20 ml of dichloromethane and 0.4 g (0.004 mol) of triethyl amine were charged in a 100 ml flas. 1.37 g (0.010 moles) of chlomethyl butyrate were slowly added at 25-30 ºC under nitrogen atmosphere. The reaction mass was stirred at 40-42 ºC for 25-30 hours. Afterwards, the mixture was cooled down to 25 ºC and washed twice with 25 ml of water. The organic and the aqueous layer were separated and the dichloromethane distilled out at 40-42 ºC under reduced pressure. 2.2 g of a yellow coloured liquid were obtained. Molar yield: 91.46 %, HPLC purity: 91.9% (Mixture of cis and trans isomers in the ratio of 60.94 and 39.06 % respectively).

### Example 9

### Preparation of crude clevidipine butyrate

60 g (0.167 mol) of benzylidine butyrate, 19.22 g (0.167 mol) methyl 3-amino crotonate and 300 ml isopropanol, were charged in 250 ml flask fitted with condenser. The reaction mass was stirred for 8-10 hours at reflux (80-82 ºC) followed by cooling at 40 ºC. Afterwards, 300 ml DM water were slowly added to the reaction mass and stirred for 3 hours at 25-30 ºC. Filtered the reaction mass. The solid was washed with a 25 ml of a 50% *iso*propanol:water solution (v/v) to obtain 100 g of wet cake. The wet cake was suspended in 100 ml of methyl cyclohexane and heated up to 100 ºC for 1 hour, followed by cooling and stirring at 25 °C for 2 hours. The product was filtered and washed with 25 ml methyl cyclohexane. A light yellow-coloured crude clevidipine butyrate was obtained after vacuum drying for 8-10 hours. Yield: 50.2 g, molar yield: 65.9% HPLC purity: 99.2%.

### Example 10

### Preparation of clevidipine butyrate

5.0 g (0.0139 mol) of benzylidine butyrate, 1.6 g (0.139 mol) methyl 3- amino crotonate and 25 ml diisopropyl ether were charged in a 100 ml flask fitted with condenser. The reaction mass was stirred for 8-10 hours at reflux (70 ºC). Afterwards, the reaction mass was cool to 20-25 ºC and stirred for 3 hours. The solid was filtered, washed with 20 ml of diisopropyl ether and dried under reduced pressure for 8-10 hours yielding 4.25 g of a light yellow coloured clevidipine butyrate (LOD < 1.0%). Molar yield: 67%, HPLC purity: 98.98%.
m.p. by DSC: 142.1 ºC
Clevidipine butyrate form B
Recrystallisation from diisopropyl ether yields the same polymorphic form, clevidipine butyrate form B.

### Example 11

### Purification of crude clevidipine butyrate

10 g of crude clevidipine butyrate and 100 ml isopropanol were charged in a 250 ml flask and stirred at reflux temperature (80-82 ºC) until a clear solution was obtained. Afterwards, the solution was cooled down to 30-40 ºC and 80 ml of DM water were slowly added and stirred for 3 hours at 25-30 ºC. The solid was filtered, washed with 20 ml of an isopropanol:water solution (50% v/v) and dried yielding 15.9 g of wet clevidipine butyrate (LOD= 44.6%). Yield (on dry basis): 8.8 g, HPLC purity: 99.49%, % Recovery: 88 %.

### Example 12

### 2^{nd} purification of clevidipine butyrate

15 g of wet clevidipine butyrate (LOD: 44.6%) and 90 ml of isopropanol were charged in a 250 ml flask. The mass was heated up to 80-82 ºC until a clear solution was obtained. 0.4 g of activated charcoal were added to the clear solution and stirred for 30 minutes at 80-82 ºC. Afterwards, the solid was filtered out, while still hot, by means of a hyflow bed. The filtrate was transferred a 250 ml flask and 80 ml of DM water were slowly added inducing the precipitation of clevidipine butyrate. The obtained mixture was stirred at 25-30 ºC for 3 hours. The precipitate was filtered and washed with 20 ml of a 50% solution of isopropanol: water (v/v). The wet cake was dried under reduced pressure for 8-10 hours at 50-55 ºC, until constant weight was achieved, yielding 6.8 g of a white solid (LOD < 0.5%). % Recovery: 81.8 %, HPLC purity: 99.62%.
m.p. by DSC: 138.8 ºC.
Clevidipine butyrate form A

### Example 13

### Purification of crude clevidipine butyrate

10 g of crude clevidipine butyrate and 100 ml acetone were charged in a 500 ml flask and stirred for 10 minutes until clevidipine butyrate is completely dissolved. 80 ml of DM water were slowly added and stirred at 25-30 ºC for 3 hours. The solid was filtered and washed with 20 ml of an acetone:water solution (50% v/v) yielding 14.6 g of wet clevidipine butyrate (LOD: 42%). Yield (on dry basis): 8.46 g, HPLC purity: 99.73%, % Recovery: 84.6%.

### Example 14

### 2^{nd} purification of clevidipine butyrate

14.6 g of wet clevidipine butyrate and 85 ml acetone were charged in a 250 ml flask and stirred until the solid was completely dissolved. 0.8 g activated charcoal were added to the reaction mass and stirred for 30 minutes at 25-30 ºC. Afterwards, the solid was filtered through a hyflow bed and the collected filtrate charged in a 250 ml flask. 68 ml of DM water were slowly added to the collected filtrate inducing the precipitation of clevidipine butyrate. The obtained mixture was stirred at 25-30 ºC for 3 hours. The solid was filtered and washed with 20 ml of a 50% solution of acetone: water (v/v). The wet cake was dried under reduced pressure at 50-55 ºC for 8-10 hours, until constant weight was achieved (LOD <0.5%). Yield 7.1 g % Recovery: 83.9 %, HPLC purity: 99.88%,
m.p. by DSC: 138.9 ºC
Clevidipine butyrate form A

### Example 15

### Purification of clevidipine butyrate

5 g of clevidipine butyrate (HPLC purity 99.88%) and 50 ml methanol were charged in a 250 ml flask. The mixture was stirred for at 55-60 ºC until a clear solution was obtained. Afterwards, the solution was cooled down to 35-40 ºC and 40 ml of DM water were slowly added. The mixture was stirred at 25-30 ºC for 3 hours. The solid was filtered and washed with 25 ml of a 50% (v/v) solution of methanol:water. The wet cake was dried under reduced pressure at 50-55 ºC for 8-10 hours to yield a 4.5 g of a white solid. HPLC purity: 99.91, % Recovery: 90%.
m.p. by DSC: 142.8 ºC
Clevidipine butyrate form B

### Example 16

### Purification of clevidipine butyrate

1 g of clevidipine butyrate (HPLC purity 99.88%) and 10 ml methanol were charged in a 100 ml flask. The mixture was stirred for at 55 -60 ºC until a clear solution was obtained. Afterwards, the solution was cooled down to 35 -40 ºC and 8 ml of DM water were slowly added. The mixture was stirred at 25-30 ºC for 3 hours. The solid was filtered and washed with 5 ml of a 50% (v/v) solution of methanol:water. The wet cake was dried under reduced pressure at 50-55 ºC for 8-10 hours to yield a 0.9 g of a white solid. HPLC purity: 99.91, % Recovery: 90%.
m.p. by DSC: 143.7 ºC
Clevidipine butyrate mixture of form A and form B

## Claims

1. A process for the preparation of clevidipine butyrate comprising the step:
(i) reacting a compound of formula (I): 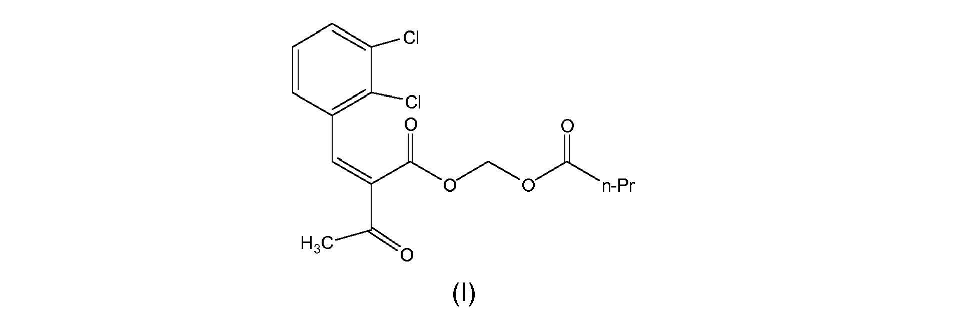 with methyl 3-amino crotonate to obtain clevidipine butyrate, wherein the step (i) is carried out in the presence of at least an organic solvent or a solvent mixture comprising an organic solvent; and
wherein the compound of formula (I) is prepared by esterification of a compound of formula (II) or a salt thereof: 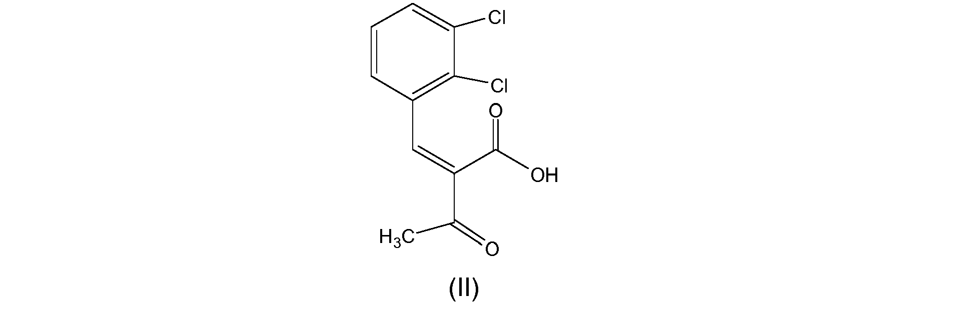 with a compound of formula L-CH₂OCO-n-Pr, wherein L is a leaving group.

2. The process according to claim 1, wherein the ratio of methyl 3-aminocrotonate to compound of formula (I) ranges from 2:1 to 1:2, preferably from 1.25:1 to 1:1.25, and more preferably is about 1:1.

3. The process according to any one of the preceding claims, wherein the step (i) is carried out at a temperature below 150 °C.

4. The process according to any one of the preceding claims, wherein the preferred organic solvent is selected form methanol, ethanol, *iso*-propyl alcohol, n-propanol, *tert*-butanol, acetone, methyl ethyl ketone, methyl *tert*-butyl ether, ethyl acetate, methyl acetate, propyl acetate, *iso*propyl acetate, butyl acetate, *iso*butyl acetate, diisopropyl ether toluene, methyl cyclohexane, xylene or mixtures thereof.

5. The process according to the preceding claim, wherein the organic solvent is ethanol, *iso*propyl alcohol, n-propyl alcohol, diisopropyl ether or mixtures thereof.

6. The process according to any one of claims 1 to 5, wherein the leaving group is a carboxyl ester, an halogen or a sulphonyloxy groups represented by -OSO₂R₂, , wherein the preferred R₂ is alkyl, p-toluoyl, trifluoromethyl or fluorine.

7. The process according to claim 6, wherein the leaving group is trifluoromethylsulfonyl, para-nitrobenzenesulfonyl, para-toluenesulfonyl, methylsulfonyl, trifluoroacetyl, para-nitrobenzoyl, para-methylbenzoyl, acetyl, iodine, bromine, chlorine or fluorine.

8. The process according to any one of the claim 6 or 7, wherein the esterification is carried out in the presence of at least one organic and/or inorganic base.

9. The process according to any one of the claims 6 to 8, wherein the compound of formula (II) is obtained by saponification of the compound of formula (III), wherein R₁ is selected from: an alkyl, preferably selected from methyl, ethyl, propyl, *iso*propyl, butyl and *tert-*butyl; and an aryl, preferably selected from trityl, phenyl and benzyl.

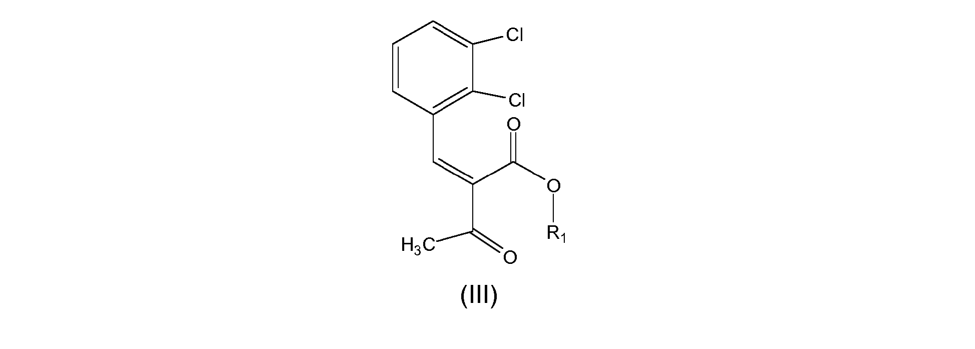

10. The process according to claim 9, wherein the compound of formula (III) is obtained by reacting 2,3-dichloro-benzaldehyde, compound of formula (IV), with CH₃-CO-CH₂-COOR₁, compound of formula (V), and by adding a base and/or an acid: 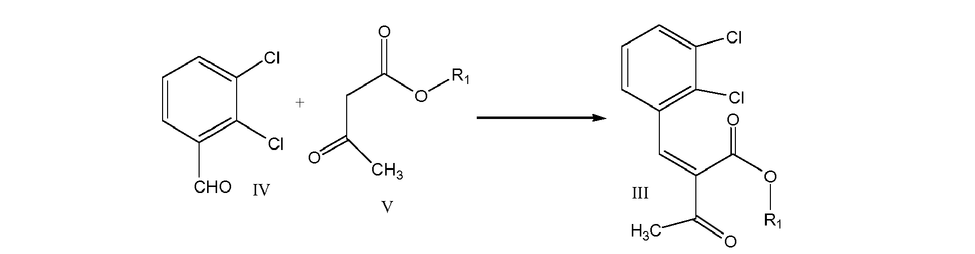 wherein R₁ is as defined above in the preceding claim.

## Patentansprüche

1. Verfahren zur Herstellung von Clevidipinbutyrat, das folgende Schritte umfasst:
(i) Umsetzen einer Verbindung der Formel (I): 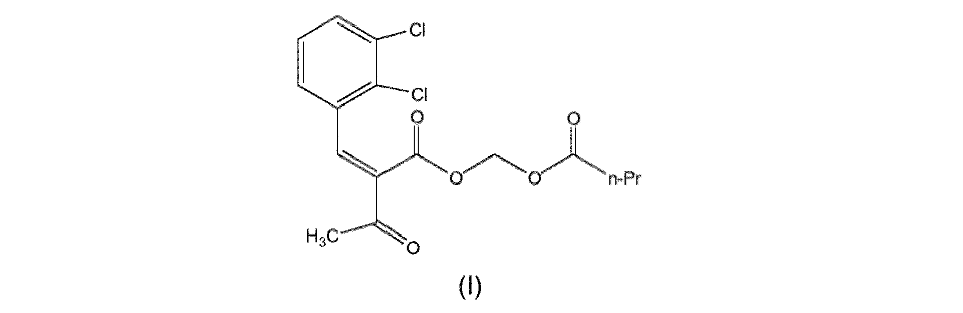 mit Methyl-3-aminocrotonat zu Clevidipinbutyrat, wobei Schritt (i) in Anwesenheit von mindestens einem organischen Lösungsmittel oder einem Lösungsmittelgemisch, das ein organisches Lösungsmittel umfasst, durchgeführt wird; und
wobei die Verbindung der Formel (I) durch Veresterung einer Verbindung der Formel (II) oder eines Salzes davon: 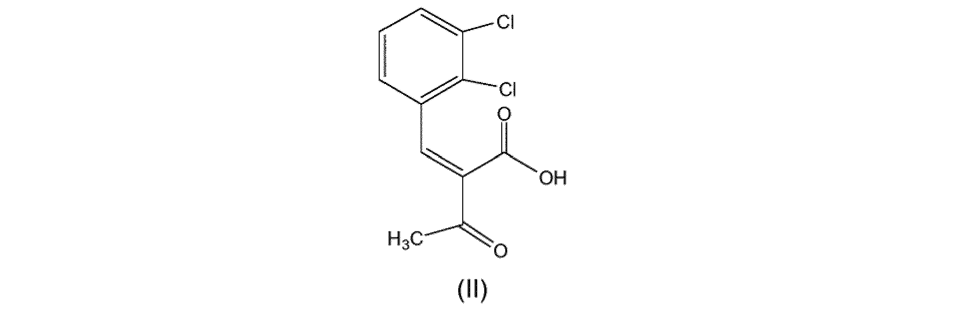 mit einer Verbindung der Formel L-CH₂OCO-n-Pr, worin L eine Abgangsgruppe ist, hergestellt wird.

2. Verfahren nach Anspruch 1, wobei das Verhältnis von Methyl-3-aminocrotonat zur Verbindung der Formel (I) zwischen 2:1 und 1:2, bevorzugt zwischen 1,25:1 und 1:1,25, liegt und besonders bevorzugt etwa 1:1 beträgt.

3. Verfahren nach irgendeinem der vorhergehenden Ansprüche, wobei Schritt (i) bei einer Temperatur unterhalb von 150 °C durchgeführt wird.

4. Verfahren nach irgendeinem der vorhergehenden Ansprüche, wobei das bevorzugte organische Lösungsmittel aus Methanol, Ethanol, iso-Propylalkohol, n-Propanol, tert-Butanol, Aceton, Methylethylketon, Methyl-tert-butylether, Ethylacetat, Methylacetat, Propylacetat, Isopropylacetat, Butylacetat, Isobutylacetat, Diisopropylethertoluol, Methylcyclohexan, Xylol oder Mischungen davon ausgewählt wird.

5. Verfahren nach dem vorhergehenden Anspruch, wobei das organische Lösungsmittel Ethanol, Isopropylalkohol, n-Propylalkohol, Diisopropylether oder Mischungen davon ist.

6. Verfahren nach irgendeinem der Ansprüche 1 bis 5, wobei die Abgangsgruppe eine Carboxylester-, eine Halogen- oder eine Sulphonyloxy-Gruppe ist, die durch -OSO₂R₂ dargestellt ist, worin R₂ vorzugsweise Alkyl, p-Toluoyl, Trifluormethyl oder Fluor ist.

7. Verfahren nach Anspruch 6, wobei die Abgangsgruppe Trifluormethylsulphonyl, para-Nitrobenzolsulphonyl, para-Toluolsulphonyl, Methylsulphonyl, Trifluoracetyl, para-Nitrobenzoyl, para-Methylbenzoyl, Acetyl, Jod, Brom, Chlor oder Fluor ist.

8. Verfahren nach irgendeinem der Ansprüche 6 oder 7, wobei die Veresterung in Anwesenheit von mindestens einer organischen und/oder anorganischen Base durchgeführt wird.

9. Verfahren nach irgendeinem der Ansprüche 6 bis 8, wobei die Verbindung der Formel (II) durch Verseifung der Verbindung der Formel (III) erhalten wird, worin R₁ ausgewählt wird aus: Alkyl, vorzugsweise ausgewählt aus Methyl, Ethyl, Propyl, Isopropyl, Butyl und tert-Butyl; und Aryl, vorzugsweise ausgewählt aus Trityl, Phenyl und Benzyl.

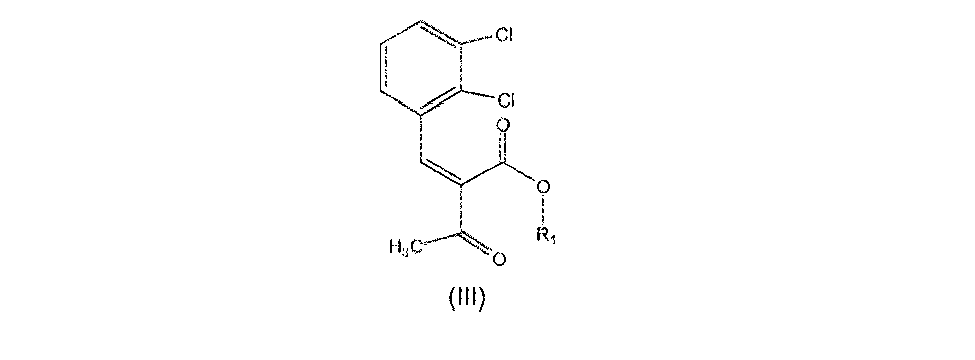

10. Verfahren nach Anspruch 9, wobei die Verbindung der Formel (III) durch Umsetzen von 2,3-Dichlorbenzaldehyd, der Verbindung der Formel (IV), mit CH₃-CO-CH₂-COOR₁, der Verbindung der Formel (V), und Zusatz einer Base und/oder einer Säure erhalten wird: 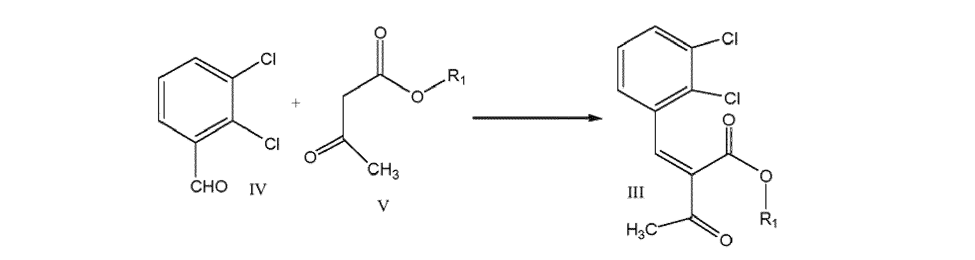 worin R₁ wie im vorhergehenden Anspruch definiert ist.

## Revendications

1. Un procédé pour la préparation de butyrate de clévidipine comprenant l'étape consistant à :
(i) faire réagir un composé de formule (I) : 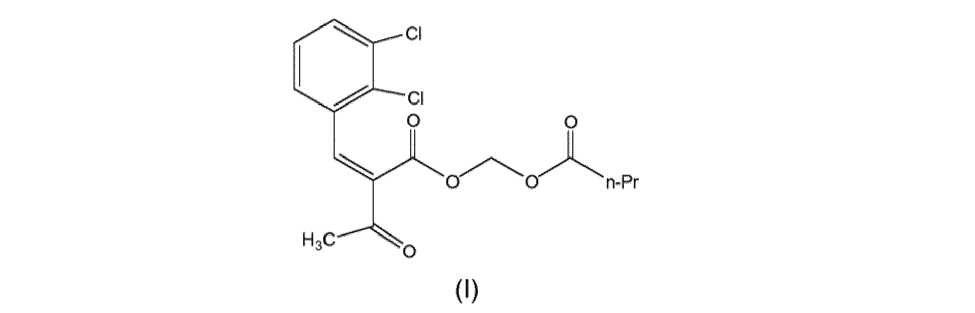 avec du méthyl 3-amino crotonate pour obtenir du butyrate de clévidipine, dans lequel l'étape (i) est réalisée en présence d'au moins un solvant organique ou d'un mélange de solvants comprenant un solvant organique ; et dans lequel le composé de formule (I) est préparé par estérification d'un composé de formule (II) ou un de ses sels : 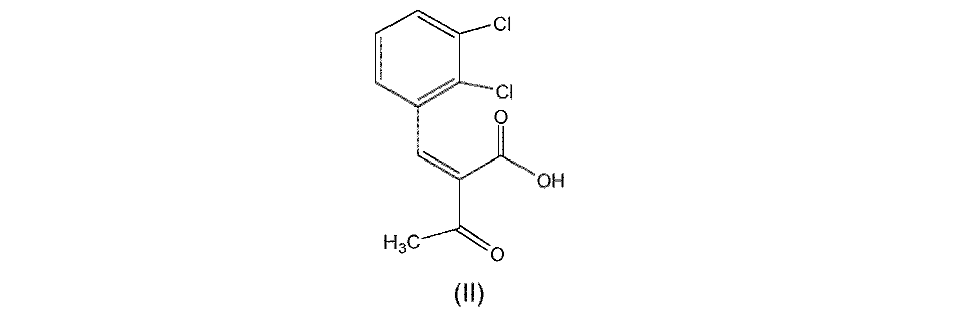 avec un composé de formule L-CH₂OCO-n-Pr, dans lequel L est un groupe partant.

2. Le procédé selon la revendication 1, dans lequel le rapport entre méthyl 3-aminocrotonate et composé de formule (I) va de 2:1 à 1:2, de préférence de 1.25:1 à 1:1.25, et de préférence encore est d'environ 1:1.

3. Le procédé selon n'importe laquelle des revendications précédentes, dans lequel l'étape (i) est réalisée à une température inférieure à 150°C.

4. Le procédé selon n'importe laquelle des revendications précédentes, dans lequel le solvant organique préféré est sélectionné parmi méthanol, éthanol, alcool isopropylique, n-propanol, tert-butanol, acétone, méthyléthylcétone, méthyl tert-butyl éther, acétate d'éthyle, acétate de méthyle, acétate de propyle, acétate d'isopropyle, acétate de butyle, acétate d'isobutyle, diisopropyle éther toluène, méthylcyclohexane, xylène ou des mélanges de ces derniers.

5. Le procédé selon la revendication précédente, dans lequel le solvant est l'éthanol, alcool isopropylique, alcool n-propylique, éther diisopropylique ou des mélanges de ces derniers.

6. Le procédé selon n'importe laquelle des revendications1 à 5, dans lequel le groupe partant est un ester carboxylique, un halogène ou un groupe sulfonyloxy représenté par -OSO₂R₂, dans lequel le R₂ préféré est alkyle, p-toluoyl, trifluorométhyle ou fluor.

7. Le procédé selon la revendication 6, dans lequel le groupe partant est trifluorométhylsulfonyl, para-nitrobenzènesulfonyl, para-toluènesulfonyl, méthylsulfonyl, trifluoroacétyl, para-nitrobenzoyl, para-méthylbenzoyl, acétyle, iode, brome, chlore ou fluor.

8. Le procédé selon n'importe laquelle des revendications 6 ou 7, dans lequel l'estérification est réalisée en présence d'au moins une base organique et/ou inorganique.

9. Le procédé selon n'importe laquelle des revendications 6 à 8, dans lequel le composé de formule (II) est obtenu par saponification du composé de formule (III), dans lequel le R₁ est sélectionné parmi : un alkyle, de préférence sélectionné parmi : méthyle, éthyle, propyle, isopropyle, butyle et tert-butyl ; et un aryle, de préférence sélectionné parmi trityle, phényle et benzyle.

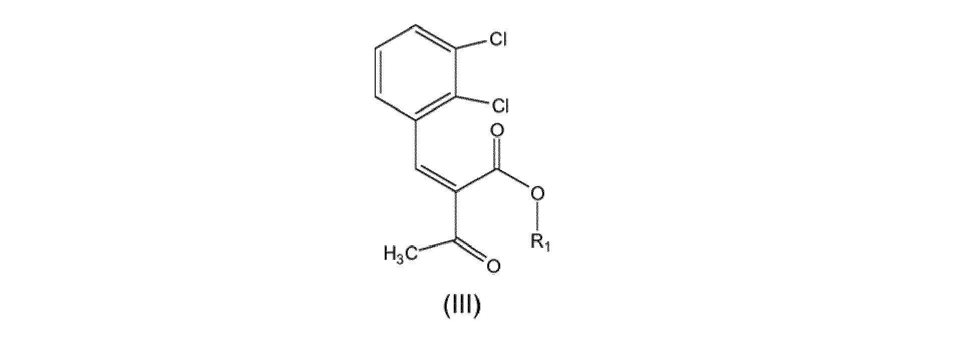

10. Le procédé selon la revendication 9, dans lequel le composé de formule (III) est obtenu en faisant réagir 2,3-dichloro-benzaldehyde, composé de formule (IV), avec CH₃-CO-CH₂-COOR₁, composé de formule (V), et en ajoutant une base et/ou un acide : 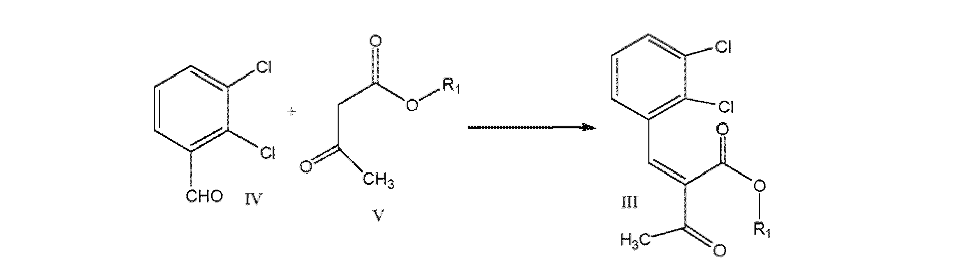 dans lequel R₁, est tel que défini dans la revendication précédente ci-dessus.
